# EUROPÄISCHE PATENTANMELDUNG

(11) **EP 1 466 881 A2**
(43) Veröffentlichungstag der Anmeldung: **13.10.2004**
(21) Anmeldenummer: 04002286.5
(22) Anmeldetag: 03.02.2004
(51) Int. Cl.: C07C 5/25, C07C 7/04, C07C 7/163, C07C 7/167, C07C 6/04, C07C 11/08, C07C 11/107

(54) **Verfahren zur Herstellung von 1-Buten**

(30) Priorität: 14.03.2003 DE 10311139
(71) Anmelder: BASF AKTIENGESELLSCHAFT, 67056 Ludwigshafen (DE)
(72) Erfinder: Sigl, Marcus, Dr., 68167 Mannheim (DE); Übler, Christoph, 67280 Quirnheim (DE)

(57) **Zusammenfassung**

Verfahren zur Herstellung eines 1-Buten-haltigen C4-Kohlenwasserstoffstroms (1-C₄⁼-Strom) aus einem 1-Buten und 2-Butenen-haltigen C4-Kohlenwasserstoffstrom (1- und 2-C₄⁼-Feedstrom), dessen Gehalt an 1-Buten geringer ist als der des 1-C₄⁼-Stroms, indem man
a) in einer Destillationskolonne den 1- und 2-C₄⁼-Feedstrom und einen mittels des nachfolgenden Schrittes (b) hergestellten 1-Buten und 2-Butenen-haltigen C4-Kohlenwasserstoffstrom (1- und 2-C₄⁼-Kreisstrom), dessen Gehalt an 1-Buten geringer ist als der des 1-C₄⁼-Stroms, einspeist und aus der Destillationskolonne den 1-C₄⁼-Strom und einen 2-Butenen-haltigen C4-Kohlenwasserstoffstrom (2-C₄⁼-Strom), dessen Gehalt an 1-Buten geringer ist als der des 1- und 2-C₄⁼-Feedstroms und des 1- und 2-C₄⁼-Kreisstroms, abzieht (Schritt a) und
b) aus dem 2-C₄⁼-Strom den 1- und 2-C₄⁼-Kreisstrom herstellt, indem man den 2-C₄⁼-Strom in einer Reaktionszone mit einem Isomerisierungskatalysator in Kontakt bringt, der die Umsetzung von 2-Butenen zu 1-Buten katalysiert (Schritt b).

## Beschreibung

Die Erfindung betrifft ein Verfahren zur Herstellung eines 1-Buten-haltigen C4-Kohlenwasserstoffstroms (1-C₄⁼-Strom) aus einem 1-Buten- und 2-Butene-haltigen C4-Kohlenwasserstoffstrom (1- und 2-C₄⁼-Feedstrom), dessen Gehalt an 1-Buten geringer ist als der des 1-C₄⁼-Stroms, indem man
a) in einer Destillationskolonne den 1- und 2-C₄⁼-Feedstrom und einen mittels des nachfolgenden Schrittes (b) hergestellten 1-Buten- und 2-Butene-haltigen C4-Kohlenwasserstoffstrom (1- und 2-C₄⁼-Kreisstrom), dessen Gehalt an 1-Buten geringer ist als der des 1-C₄⁼-Stroms, einspeist und aus der Destillationskolonne den 1-C₄⁼-Strom und einen 2-Butene-haltigen C4-Kohlenwasserstoffstrom (2-C₄⁼-Strom), dessen Gehalt an 1-Buten geringer ist als der des 1- und 2-C₄⁼-Feedstroms und des 1- und 2-C₄⁼-Kreisstroms, abzieht (Schritt a) und
b) aus dem 2-C₄⁼-Strom den 1- und 2-C₄⁼-Kreisstrom herstellt, indem man den 2-C₄⁼-Strom in einer Reaktionszone mit einem Isomerisierungskatalysator in Kontakt bringt, der die Umsetzung von 2-Butenen zu 1-Buten katalysiert (Schritt b).

Es ist allgemein bekannt, dass die Isomerisierung von 2-Butenen nach 1-Buten eine Gleichgewichtsreaktion ist. Cis-2-Buten, trans-2-Buten und 1-Buten liegen im Gleichgewicht nebeneinander vor. Die thermodynamischen Daten sind in D. Stull, "The Chemical Thermodynamics of Organic Compounds", J. Wiley, New York 1969 aufgeführt.

Aus EP-A-751106 ist es bekannt, aus einem C4-Kohlenwasserstoffstrom Buten-1 zu gewinnen, indem man
a) den Butene enthaltenden Kohlenwasserstoffstrom einer Selektivhydrierung unterwirft, um mehrfach ungesättigte Kohlenwasserstoffe zu beseitigen,
b) den gemäß Schritt (a) erhaltenen Kohlenwasserstoffstrom fraktioniert destilliert, wobei man eine Fraktion an reinem 1-Buten erhält sowie eine Fraktion enthaltend Paraffine und 2-Butene,
c) aus der Fraktion, enthaltend Paraffine, 1-Buten und 2-Butene, die Paraffine durch Behandlung mit einem Molekularsieb entfernt,
d) die gemäß Schritt c) erhaltene Fraktion einer Doppelbindungsisomerisierung unterwirft,
e) die der Isomerisierung gemäß Schritt d) unterworfene Fraktion in Schritt a) zurückführt, nachdem sie mit einem frischem C4-Kohlenwasserstoff versetzt wurde.

Der wesentliche Unterschied zum erfindungsgemäßen Verfahren liegt darin, dass die der Isomerisierung gemäß Schritt d) unterworfene Fraktion in den Hydrierungsschritt a) zurückgeführt wird und nicht unmittelbar in die Destillationsschritt. Nachteilig hieran ist, dass das Volumen des Kreisstroms aufgebläht wird und der Reaktor, in dem die Hydrierung durchgeführt wird, in hohem Maße mit gegenüber der Hydrierung inerten Verbindungen belastet wird, die erst in der folgenden Destillation entfernt werden.

In der WO 02/096843 ist ein Verfahren zur Gewinnung von 1-Buten aus 2-Butenen beschrieben. Hierbei wird ein hauptsächlich 2-Butene enthaltender Kohlenwasserstoffstrom zuerst einer Isomerisierung unterzogen und die dabei gebildete Reaktionsmischung einer Destillation unterworfen. Bei der Destillation wird ein 1-Buten-reicher Strom von einem 2-Butene-reichen Strom getrennt und letzterer in die Isomerisierungseinheit zurückgeführt. Dieses Verfahren ist jedoch für einen Kohlenwasserstoffstrom, der signifikante Mengen an 1-Buten enthält unwirtschaftlich. Zudem werden bedingt durch die Anwesenheit von mehrfach ungesättigten Verbindungen im Isomerisierungsschritt relativ viele unerwünschte Nebenprodukte gebildet. Durch die Durchführung der Destillation im Anschluss an die Isomerisierungsstufe gelangen störende leichtflüchtige Bestandteile des Feeds (z.B. Butin, Butadiene, Propadien, Propin) in den Isomerisierungsreaktor und können dort den Katalysator schädigen oder zur Bildung unerwünschter Nebenprodukte führen. Durch die vorgeschaltete Destillation im erfindungsgemäßen Verfahren werden diese leichtflüchtigen Komponenten großteils abgetrennt und gelangen nicht in den 2-C₄⁼-Strom zum Isomerisierungsreaktor.

Aufgabe der vorliegenden Erfindung war es deshalb, ein Verfahren bereitzustellen, mit dem man in besonders wirtschaftlicher Weise den 1-Buten-Anteil auf Kosten des 2-Butene-Anteils in C4-Kohlenwasserstoffströmen erhöhen kann.

Bei dem 1 - und 2-C₄⁼-Feedstrom handelt es sich um C₄-Schnitte, die im Allgemeinen einen Gehalt an Butenen von 30 bis 100, bevorzugt 40 bis 98, besonders bevorzugt 50 bis 95 Gew.-% aufweisen. Neben den Butenen können im 1- und 2-C₄⁼-Feedstrom noch bis zu 10, bevorzugt bis zu 5 Gew.-% mehrfach ungesättigte Verbindungen oder Alkine, vor allem solche mit 3- oder 4-Kohlenstoffatomen wie Butadiene, Butine, Vinylacetylen, Propin und Propadien enthalten sein. Weiterhin können noch 0,5 bis 60, bevorzugt 1 bis 50 Gew.-% C4-Alkane und Iso-Buten enthalten sein. Weitere Kohlenwasserstoffe mit mehr als 5 Kohlenstoffatomen, insbesondere Pentane und Pentene sind ggf. in Mengen bis maximal 10 Gew.-% enthalten.

Der Oberbegriff "Butene" wird in diesem Text nur für lineare Butene verwendet und umfasst nicht Iso-Buten.

Insbesondere eignen sich sog. Raffinate (Raffinat I oder II).

Solche Raffinate I sind herstellbar, indem man
- Naphtha oder sonstige Kohlenwasserstoffverbindungen einem Steamcracking- oder FCC-Prozess unterwirft und aus dem dabei gebildeten Stoffstrom eine C₄-Kohlenwasserstofffraktion abzieht
- aus der C₄-Kohlenwasserstofffraktion einen im wesentlichen aus Isobuten, 1-Buten, 2-Butenen und Butanen bestehenden C₄-Kohlenwasserstoffstrom (Raffinat I) herstellt, indem man mittels Selektivhydrierung die Butadiene und Butine zu Butenen oder Butanen hydriert oder die Butadiene und Butine durch Extraktivdestillation entfernt.

Weiterhin sind die Raffinate I erhältlich, indem man
- aus einem Butane enthaltenden Kohlenwasserstoffstrom durch Dehydrierung und nachfolgende Isolierung der C₄-Olefine eine C₄-Olefin-Mischung herstellt
- aus der C₄-Olefin-Mischung einen im wesentlichen aus Isobuten, 1-Buten, 2-Butenen und Butanen bestehenden C4-Kohlenwasserstoffstrom (Raffinat I) herstellt, indem man mittels Selektivhydrierung die Butadiene und Butine zu Butenen oder Butanen hydriert oder die Butadiene und Butine durch Extraktivdestillation entfernt.

Aus dem Raffinat I kann das Raffinat II hergestellt werden, indem man aus dem Raffinat I den wesentlichen Anteil des Iso-Butens durch bekannte chemische, physikalischchemische oder physikalische Methoden abtrennt.

Nach einer 3. Methode lässt sich Raffinat II erhalten, indem man aus Methanol durch Dehydrierung eine C₄-Olefin-Mischung herstellt (MTO-Verfahren) und diese ggf. durch Destillation, Partialhydrierung oder Extraktivdestillation von Butadienen oder Alkinen befreit.

Zur weiteren Reinigung kann das Raffinat II durch Behandlung mit Adsorbermaterialien von Katalysatorgiften befreit werden.

Die Isomerisierung von 2-Butenen zu 1-Buten ist durch das thermodynamische Gleichgewicht der n-Buten-Isomeren begrenzt. Der Anteil an 1-Buten im thermodynamsichen Gleichgewicht wird durch hohe Temperaturen begünstigt. Die maximal erzielbaren Ausbeuten an 1-Buten (2-Butene-Umsatz x Selektivität) sind bei einem einzigen Reaktordurchgang durch das thermodynamische Gleichgewicht bei einer Temperatur von 200°C auf etwa 14 % und bei einer Temperatur von 500°C auf etwa 29 % begrenzt. Die angegebenen Ausbeuten beruhen auf den thermodynamischen Daten, die in D. Stull "The Chemical Thermodynamics of Organic Compounds", J. Wiley, New York, 1969, veröffentlicht sind. Das erfindungsgemäße Verfahren arbeitet deshalb besonders wirtschaftlich bei einem 1 - und 2-C₄⁼-Feedstrom, dessen Gehalt an 1-Buten höher ist als die Gleichgewichtskonzentration von 1-Buten bei Temperaturen von 100 bis 700, bevorzugt von 200 bis 500°C. Deshalb setzt man im Allgemeinen 1 - und 2-C₄⁼-Feedströme ein, bei denen das Verhältnis 2-Butene zu 1-Buten 6 : 1 bis 0,1 : 1, bevorzugt 3 : 1 bis 0,2 : 1 beträgt.

Die Destillation findet in einem dafür geeigneten Apparat statt, z.B. einer Glockenbodenkolonne, Füllkörperkolonne, Packungskolonne oder Trennwandkolonne. Bevorzugt ist die Destillationskolonne mit 30 bis 80, besonders bevorzugt 40 bis 75 theoretischen Trennstufen ausgeführt. Das Rücklaufverhältnis beträgt im Allgemeinen 10 bis 50. Die Destillation wird im Allgemeinen bei einem Druck von 5 bis 20 bar durchgeführt.

Bedingt durch den niedrigen Siedepunkt des 1-Butens im Vergleich zu den 2-Butenen wird man den 1-C₄⁼-Strom im oberen Teil der Kolonne abziehen, bevorzugt am Kopf der Kolonne. Der Gehalt an 1-Buten, bezogen auf die Summe an 1-Buten und 2-Butenen im 1-C₄⁼-Strom, beträgt üblicherweise 80 bis 99,99 %.

Besonders bevorzugt besteht der 1-C₄⁼-Strom aus 60 bis 99,9 Gew.-% 1-Buten und 2-Butenen, 0,01 bis 10 Gew.-% mehrfach ungesättigten Verbindungen, z.B. Butadiene, sowie 0,01 bis 40 Gew.-% Verbindungen, ausgewählt aus der Gruppe Isobutan, n-Butan und Iso-Buten.

Die mehrfach ungesättigten Verbindungen können zum einen aus dem 1- und 2-C₄⁼-Strom stammen, zum anderen werden sie unter bestimmten Bedingungen, insbesondere bei der Wahl bestimmter Katalysatoren, auch in Schritt b) gebildet.

Im 1-C₄⁼-Strom ist der Gehalt an 2-Butenen, bezogen auf den 1- und 2-C4⁼-Feedstrom, um 20 bis 99,99 % reduziert.

Zweckmäßigerweise zieht man den 2-C₄⁼-Strom im unteren Teil der Destillationskolonne, bevorzugt im unteren Fünftel der Destillationskolonne ab, besonders bevorzugt am Kolonnensumpf oder bis maximal 5 theoretischen Böden oberhalb.

Üblicherweise beträgt der Gehalt an 2-Butenen, bezogen auf die Summe aus 2-Butenen und 1-Buten im 2-C₄⁼-Strom, 85 bis 99,9%.

Der Gehalt an 2-Butenen im 1- und 2-C₄⁼-Kreistrom ist, bezogen auf seinen Gehalt im 2-C₄⁼-Strom, üblicherweise um 5 bis 30 % erniedrigt.

Um einer Anreicherung von hochsiedenden Komponenten, z.B. n-Butan und Kohlenwasserstoffverbindungen mit 5 und mehr C-Atomen, im 1- und 2-C₄⁼-Kreisstrom zu vermeiden, wird es im Allgemeinen erforderlich sein, am Sumpf der Destillationskolonne einen Teilstrom abzuziehen, der im wesentlichen aus 1-Buten, 2-Butenen, n-Butanen und Kohlenwasserstoffen mit 5 und mehr Kohlenstoffatomen besteht. Es ist jedoch gleichfalls möglich, zu diesem Zweck lediglich einen Teil des 2-C₄⁼-Stroms auszuschleusen. In diesem Fall wird der 2-C₄⁼-Strom am Sumpf abgezogen.

Üblicherweise beträgt der Gehalt an 2-Butenen, bezogen auf die Summe an 2-Butenen und 1-Buten im C₄⁺-Sumpfstrom, 90 bis 99,9 %.

Der Gehalt an 2-Butenen im C₄⁺-Sumpfstrom ist, bezogen auf seinen Gehalt im 2-C₄⁼-Strom, üblicherweise um bis zu 10 % erhöht. Die Größe des C₄⁺-Sumpfstroms und sein Gehalt an 2-Butenen richtet sich nach dem Gesamtumsatz der Umsetzung von 2-Butenen zu 1-Buten, der im Allgemeinen, bezogen auf den Gehalt an 2-Butenen im 1- und 2-C₄⁼-Feedstrom, 70 bis 99 % beträgt.

Gemäß Schritt b) wird der 2-C₄⁼-Strom über einen üblichen Isomerisierungskatalysator geleitet. Bei der Wahl des Isomerisierungskatalysators ist man nicht weiter eingeschränkt, er muss nur in der Lage sein, die Isomerisierung von 2-Butenen zu 1-Buten zu bewirken. Beispielsweise kommen hierfür basische Katalysatoren oder Katalysatoren auf Zeolithbasis zum Einsatz, daneben kann die Isomerisierung auch unter hydrierenden Bedingungen an Edelmetall-haltigen Kontakten erfolgen.

Als Katalysatoren eignen sich Erdalkalioxide auf Aluminiumoxid, die in der EP-A 718036 beschrieben sind, gemischte Aluminiumoxid/Siliziumoxidträger, die mit Oxiden der Erdalkalimetalle, Borgruppenmetalle, Lanthaniden oder Elementen der Eisengruppe dotiert sind (US 4814542), oder mit Alkalimetallen belegtes γ-Aluminiumoxid, das in der JP 51-108691 beschrieben ist. Weiterhin eignen sich Katalysatoren aus Manganoxid auf Aluminiumoxid, beschrieben in US 4289919, Katalysatoren aus Magnesium-, Alkali- und Zirkonoxiden dispergiert auf einem Aluminiumoxidträger, beschrieben in EP-A 234498 und Aluminiumoxidkatalysatoren, die zusätzlich Natriumoxid und Siliziumoxid enthalten, beschrieben in US 4229610.

Geeignete zeolithbasierte Katalysatoren sind beschrieben in EP-A 129899 (Zeolithe des Pentasil-Types). Geeignet sind weiterhin mit Alkali- oder Erdalkalimetallen ausgetauschten Molsiebe (beschrieben in US 3475511), Alumosilikate (beschrieben in US 4749819) sowie Zeolithe in Alkali- oder Erdalkaliform (beschrieben in US 4992613) und solche auf Basis kristalliner Borosilikate (beschrieben in US 4499326).

Die Katalysatoren werden üblicherweise im Festbett-, Wirbelbett- oder Wanderbett verwendet. Im praktischen Betrieb hat es sich herausgestellt, dass die Menge des 2-C₄⁼-Stroms, die pro Zeiteinheit über den Katalysator geleitet wird, 0,1 bis 40 g (2-C₄⁼-Strom)/[g (Katalysator) h] beträgt.

Für die Isomerisierung ist ein kontinuierlich durchströmtes Festbett-Reaktorsystem bevorzugt. Geeignete Reaktoren sind Rohrreaktoren, Rohrbündelreaktoren, Hordenreaktoren, Wickelreaktoren oder Wendelreaktoren. Die Umsetzung ist endotherm. Die Temperaturkontrolle kann wie üblich durchgeführt werden. Zudem kann die Reaktion auch in einem adiabaten Reaktionssystem ausgeführt werden.

Der 2-C₄⁼-Strom kann aus der Kolonne gasförmig oder flüssig abgezogen werden. Ist der 2-C₄⁼-Strom flüssig, so muss er vor der Reaktion verdampft werden. Der für die Verdampfung verwendete Apparat unterliegt dabei keiner Beschränkung. Es eignen sich übliche Verdampfertypen wie Naturumlaufverdampfer oder Zwangsumlaufverdampfer.

Bevor der gasförmige 2-C₄⁼-Strom in die Reaktionszone gemäß Schritt b) gelangt, muss er auf Reaktionstemperatur überhitzt werden. Zum Aufheizen können die üblicherweise verwendeten Apparate verwendet werden, z.B. Plattenwärmeüberträger oder Rohrbündelwärmeüberträger.

Die Isomerisierung wird bei einer Temperatur durchgeführt, bei der eine Verschiebung der Doppelbindung gewährleistet ist, Crackprozesse, Skelettisomerisierungen, Dehydrierungen und Oligomerisierungen, hingegen weitestgehend vermieden werden. Die Reaktionstemperatur liegt deshalb im Allgemeinen bei 100 bis 700, bevorzugt 200 bis 600, besonders bevorzugt bei 200 bis 500°C. Der Druck wird so eingestellt, dass der 2-C₄⁼-Strom gasförmig vorliegt. Er beträgt im Allgemeinen 0,1 bis 40, bevorzugt 1 bis 30, besonders bevorzugt 3 bis 20 bar.

Der mittels der vorstehend beschriebenen Isomerisierung hergestellte 1- und 2-C₄⁼-Kreisstrom wird an geeigneter Stelle in die Destillationskolonne zurückgeführt. Die Einspeisung in die Kolonne kann gasförmig oder flüssig erfolgen. Ist der Temperaturunterschied Reaktorausgang - Kolonnentemperatur auf Höhe der Wiedereinspeisung groß, kann es sinnvoll sein, den Reaktoraustrag abzukühlen. Die Abkühlung bzw. Kondensation erfolgt nach allgemeinen üblichen Methoden.

In einer speziellen Ausführungsform sind die Wärmeströme zum Verdampfen und Aufheizen mit den Wärmeströmen zum Abkühlen und Kondensieren kombiniert. Durch eine solche Wärmeintegration ist es möglich, den Energieverbrauch für die Reaktionseinheit zu minimieren.

Auf dem für die Reaktion eingesetzten Isomerisierungskatalysator können sich mit der Zeit Kohlenstoff-haltige Verbindungen ablagern, die zu einer Deaktivierung des Katalysators führen können. Durch ein Abbrennen dieser Ablagerungen ist es möglich, die Aktivität des Katalysators wieder zu erhöhen. Der Abbrennvorgang kann dabei in einem separaten Apparat oder bevorzugt in dem für die Reaktion verwendeten Apparat erfolgen. In einer speziellen Ausführungsform wird der Reaktor doppelt ausgelegt, so dass abwechselnd ein Apparat für die Reaktion zur Verfügung steht und in dem anderen Apparat die Regeneration erfolgt. Für den Abbrennvorgang wird der Katalysator mit einer Stickstoff/Sauerstoffmischung angeströmt. Das Volumenverhältnis Stickstoff zu Sauerstoff liegt dabei zwischen 1 und 20 Vol.-% Sauerstoff. Der Sauerstoffgehalt der Mischung kann sich während des Regenerationsvorgangs ändern. Typischerweise wird mit einem geringen Sauerstoff-Gehalt begonnen, der dann erhöht wird. Dadurch ist eine Kontrolle der durch den exothermen Abbrennvorgang entstehenden Wärmemenge möglich. Die Regeneration wird bei erhöhter Temperatur durchgeführt, typischer bei 300 bis 900°C, bevorzugt bei 350 bis 800°C, besonders bevorzugt bei 400 bis 700°C.

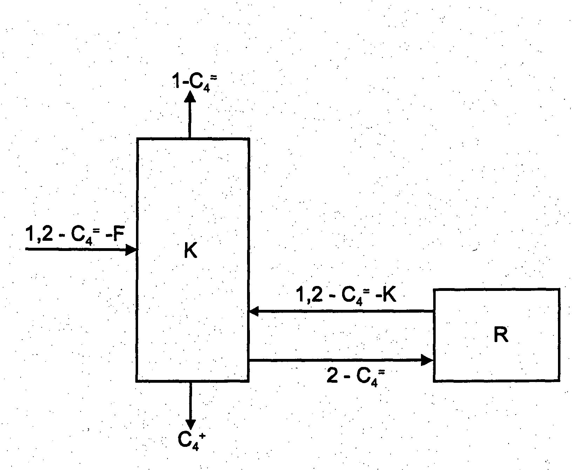

Figur 1 zeigt schematisch eine Vorrichtung zur Durchführung des erfindungsgemäßen Verfahrens unter Verwendung einer Kolonne (K) und einen Reaktor mit einem Isomerisierungskatalysator im Festbett (R). In die Kolonne (K) wird der 1- und 2-C₄⁼-Feedstrom (1,2-C₄⁼-F) eingeleitet. Am Kopf wird der 1-C₄⁼-Strom (1-C₄⁼) und am Sumpf der Strom C₄⁺ abgezogen. Als Seitenstrom wird der Kolonne der 2-C₄⁼-Strom (2-C₄⁼ ) entnommen und in den Reaktor (R) geleitet. Das dort gebildete Reaktionsgemisch, der 1,2-C₄-Kreisstrom (1,2-C₄⁼-K), wird dem Reaktor R entnommen und in die Kolonne (K) zurückgeleitet.

Der 1-C₄⁼-Strom eignet sich insbesondere für die Herstellung von 3-Hexen durch Metathese. Hierzu wird der 1-C₄⁼-Strom bei einer Temperatur von 20 bis 350°C mit einem üblichen Metathesekatalysator in Kontakt gebracht. Solche Metathesekatalysatoren sind allgemein bekannt und z.B. in der EP-A-1134271 beschrieben. Dabei handelt es sich im allgemeinen um Verbindungen eines Metalls der VIb, VIIb oder VIII-Nebengruppe des Periodensystems der Elemente enthaltenen Verbindungen.

Sofern der 1-C₄⁼-Strom Alkine oder mehrfach ungesättigte Verbindungen enthält, so empfiehlt es sich, den 1-C₄⁼-Strom von den Verbindungen zu befreien, indem man ihn in Gegenwart eines Palladiumenthaltenden Katalysators einer Selektivhydrierung unterzieht, bei der eine Umsetzung von 1-Buten zu 2-Butenen praktisch unterbleibt. Eine solche selektive Hydrierung unter Vermeidung der Isomerisierung kann erreicht werden, indem man den 1-C₄⁼-Strom bei 40 bis 60°C und einem Wasserstoffpartialdruck von 0,5 bis 10⁶ Pascal mit einem Katalysatorbett aus einem Palladium-Trägerkatalysator in Kontakt bringt. Diese Art der Hydrierung ist allgemein bekannt und beispielsweise in der Monographie Petrochemical Processes, Volume 1, Synthesis-Gas Derivates and Major Hydrocarbons, A. Chauvel, G. Lefebvre, L. Castex, Institut Francais du Petrol Publications, von 1989, Editions Technip, 27 Rue Ginoux, 75737 Paris, Cedex 15, auf Seiten 208 und 209 beschrieben.

Ein nach dem oben beschriebenen Verfahren hergestellter 1-Buten reicher C4-Strom kann weiterhin für eine Vielzahl von Reaktionen als Ausgangsstoff eingesetzt werden. Beispielhaft seinen genannt: Dimerisierung, Oligomerisierung, Epoxidation, Carbonylierung und Copolymerisation mit Ethylen.

### Experimenteller Teil

### Beispiel 1

Zur Durchführung des Experiments dient eine Vorrichtung gemäß Figur 1. Es wird eine Glockenbodenkolonne (K) mit 85 Böden und 55 mm Innendurchmesser verwendet. Der Druck in der Kolonne beträgt 8 bar, die Sumpftemperatur liegt bei 71 °C, die Kopftemperatur bei 63°C. Der Zulauf an 1,2-C₄⁼-F erfolgt auf Höhe Boden 55, 1-C₄⁼ wird am Kopf der Kolonne abgezogen, der 2-Butene-reiche Strom (2-C₄⁼) wird auf Boden 5 abgezogen und in den Isomerisierungsreaktor (R) geleitet. Der den Isomerisierungsreaktor (R) verlassende Feed (1,2-C₄⁼-K) wird auf Höhe des Bodens 35 zurück in die Kolonne geleitet. Eine hochsiedende Fraktion (C₄⁺) wird am Sumpf der Kolonne abgezogen. Die Menge und Zusammensetzung der einzelnen Ströme ist in Tabelle 1 dargestellt. Die Isomerisierung wird bei 250°C und 6 bar Druck an einem Zeolith-Katalysator (Na-ZBM-11) durchgeführt. Das Katalysatorvolumen beträgt 3 dm³.

**Tabelle 1**

| Strom | Einheit | 1,2-C₄⁼-F | 1-C₄⁼ | C₄⁺ | 2-C₄⁼ | 1,2-C₄⁼-K |
|---|---|---|---|---|---|---|
| von | --- | --- | K | K | K | R |
| nach | --- | K | --- | --- | R | K |
| Menge | kg/h | 0,650 | 0,607 | 0,043 | 3,259 | 3,259 |
| iso-Butan | g/g | 0,0250 | 0,0268 | 0,0000 | 0,0000 | 0,0000 |
| n-Butan | g/g | 0,1250 | 0,1154 | 0,2984 | 0,3105 | 0,3110 |
| 1-Buten | g/g | 0,4150 | 0,7922 | 0,0348 | 0,0427 | 0,1078 |
| c-Buten-2 | g/g | 0,2075 | 0,0027 | 0,2814 | 0,2631 | 0,2259 |
| t-Buten-2 | g/g | 0,2075 | 0,0390 | 0,3797 | 0,3830 | 0,3539 |
| iso-Buten | g/g | 0,0200 | 0,0214 | 0,0001 | 0,0001 | 0,0002 |
| Dichte | kg/m³ | 594 | 535 | 535 | 8 | 8 |
| Temperatur | °C | 25,0 | 62,7 | 71,4 | 250,0 | 245,2 |
| Druck | bar | 8,5 | 8,0 | 8,2 | 6,0 | 6,0 |

### Beispiel 2

Der Versuch wird analog Beispiel 1 durchgeführt, mit dem Unterschied, dass der Isomerisierungsreaktor bei 400°C und einem basischen Katalysator (SrO auf γ-Al₂O₃, 4,2 Gew.-% Sr) betrieben wird, der Feed aus einer Butane-/ Butene-Trennung stammt und das Kopfprodukt mit einer Reinheit von 99,9 Gew.-% 1-Buten/∑2-Butene gewonnen wird. Für diesen Fall ist eine Glockenbodenkolonne (K) mit 115 Böden und 55 mm Innendurchmesser zu verwenden. Der Druck in der Kolonne beträgt 8 bar, die Sumpftemperatur liegt dann bei 71 °C, die Kopftemperatur bei 63°C. Der Zulauf an 1,2-C₄⁼-F erfolgt auf Höhe Boden 42, 1-C₄⁼ wird am Kopf der Kolonne abgezogen, der 2-Butene-reiche Strom (2-C₄⁼) auf Boden 5 abgezogen und in den Isomerisierungsreaktor (R) geleitet. Der den Isomerisierungsreaktor (R) verlassende Feed (1,2-C₄⁼-K) wird auf Höhe des Bodens 27 zurück in die Kolonne geleitet. Eine hochsiedende Fraktion (C₄⁺) wird am Sumpf der Kolonne abgezogen. Die Massenströme und Zusammensetzungen der einzelnen Ströme sind in Tabelle 2 dargestellt.

**Tabelle 2**

| Strom | Einheit | 1,2-C₄⁼-F | 1-C₄⁼ | C₄⁺ | 2-C₄⁼ | 1,2-C₄⁼-K |
|---|---|---|---|---|---|---|
| von | --- | --- | K | K | K | R |
| nach | --- | K | --- | --- | R | K |
| Menge | kg/h | 0,650 | 0,621 | 0,029 | 3,236 | 3,236 |
| iso-Butan | g/g | 0,000 | 0,0000 | 0,000 | 0,000 | 0,000 |
| n-Butan | g/g | 0,0300 | 0,0241 | 0,261 | 0,274 | 0,275 |
| 1-Buten | g/g | 0,3230 | 0,9702 | 0,036 | 0,045 | 0,166 |
| c-Buten-2 | g/g | 0,2920 | 0,0000 | 0,309 | 0,291 | 0,236 |
| t-Buten-2 | g/g | 0,3550 | 0,0010 | 0,383 | 0,389 | 0,321 |
| iso-Buten | g/g | 0,0000 | 0,0000 | 0,000 | 0,000 | 0,000 |
| Dichte | kg/m³ | 601 | 537 | 537 | 6 | 6 |
| Temperatur | °C | 25,0 | 62,3 | 71,6 | 400,0 | 392 |
| Druck | bar | 8,5 | 8,0 | 8,2 | 6,0 | 6,0 |

## Patentansprüche

1. Verfahren zur Herstellung eines 1-Buten-haltigen C4-Kohlenwasserstoffstroms (1-C₄⁼-Strom) aus einem 1-Buten- und 2-Butene-haltigen C4-Kohlenwasserstoffstrom (1- und 2-C₄⁼-Feedstrom), dessen Gehalt an 1-Buten geringer ist als der des 1-C₄⁼-Stroms, indem man
a) in einer Destillationskolonne den 1- und 2-C₄⁼-Feedstrom und einen mittels des nachfolgenden Schrittes (b) hergestellten 1-Buten- und 2-Butene-haltigen C4-Kohlenwasserstoffstrom (1- und 2-C₄⁼-Kreisstrom), dessen Gehalt an 1-Buten geringer ist als der des 1-C₄⁼-Stroms, einspeist und aus der Destillationskolonne den 1-C₄⁼-Strom und einen 2-Butene-haltigen C4-Kohlenwasserstoffstrom (2-C₄⁼-Strom), dessen Gehalt an 1-Buten geringer ist als der des 1 - und 2-C₄⁼-Feedstroms und des 1 - und 2-C₄⁼-Kreisstroms, abzieht (Schritt a) und
b) aus dem 2-C₄⁼-Strom den 1- und 2-C₄⁼-Kreisstrom herstellt, indem man den 2-C₄⁼-Strom in einer Reaktionszone mit einem Isomerisierungskatalysator in Kontakt bringt, der die Umsetzung von 2-Butenen zu 1-Buten katalysiert (Schritt b).

2. Verfahren nach Anspruch 1, wobei man einen 1- und 2-C₄⁼-Feedstrom einsetzt, bei dem das Verhältnis 2-Butene zu 1-Buten 6 : 1 bis 0,1 : 1 beträgt.

3. Verfahren nach einem der vorstehenden Ansprüche, wobei man einen 1- und 2-C₄⁼-Feedstrom einsetzt, der maximal 5 Gew.-% mehrfach ungesättigte Verbindungen oder Alkine enthält.

4. Verfahren nach einem der vorstehenden Ansprüche, wobei man einen 1- und 2-C₄⁼-Feedstrom einsetzt, bei dem der Gehalt an Butenen 30 bis 100 Gew.-% beträgt.

5. Verfahren nach einem der vorstehenden Ansprüche, wobei man die Destillationskolonne mit 30 bis 80 theoretischen Trennstufen ausführt und mit einem Rücklaufverhältnis von 10 bis 50 betreibt.

6. Verfahren nach einem der vorstehenden Ansprüche, wobei der Gehalt an 1- Buten, bezogen auf die Summe an 1-Buten und 2-Butenen im 1-C₄⁼-Strom, 80 bis 99,99 % beträgt.

7. Verfahren nach einem der vorstehenden Ansprüche, wobei der 1-C₄⁼-Strom besteht aus
- 60 bis 99,99 Gew.-% 1-Buten und 2-Butenen und
- 0,01 bis 40 Gew.-% Verbindungen, ausgewählt aus der Gruppe Butadiene, iso-Butan, n-Butan und iso-Buten.

8. Verfahren nach einem der vorstehenden Ansprüche, wobei man den 2-C₄⁼-Strom im unteren Fünftel der Destillationskolonne abzieht.

9. Verfahren nach einem der vorstehenden Ansprüche, wobei der Gehalt an 2-Butenen im 1- und 2-C₄⁼-Kreisstrom, bezogen auf seinen Gehalt im 2-C₄⁼-Strom, um 5 bis 30 % erniedrigt ist.

10. Verfahren nach einem der vorstehenden Ansprüche, wobei man am Sumpf der Destillationskolonne einen Teilstrom (C₄⁺) abzieht, der im wesentlichen aus 1-Buten, 2-Butenen, n-Butan und Kohlenwasserstoffen mit 5 und mehr Kohlenstoffatomen besteht.

11. Verfahren nach einem der vorstehenden Ansprüche, wobei die Temperatur in der Reaktionszone von Schritt b 200 bis 500°C und der Druck 1 bis 20 bar beträgt.

12. Verfahren nach einem der vorstehenden Ansprüche, wobei der Umsatz an 2-Butenen zu 1-Buten, bezogen auf den Gehalt an 2-Butenen im 1- und 2-C₄⁼-Feedstrom, 70 bis 99 % beträgt.

13. Verfahren nach einem der vorstehenden Ansprüche, wobei man aus dem 1-C-₄⁼-Strom einen 3-Hexen enthaltenden Strom herstellt, indem man den 1 -C₄⁼-Strom bei einer Temperatur von 20 bis 350°C mit einem Metathesekatalysator in Kontakt bringt.

14. Verfahren nach einem der vorstehenden Ansprüche, wobei man den 1-C₄⁼-Strom von mehrfach ungesättigten Verbindungen und Alkinen befreit, indem man ihn in Gegenwart eines Palladium enthaltenden Katalysators einer Selektivhydrierung unterzieht, bei der eine Umsetzung von 1-Buten zu 2-Butenen praktisch unterbleibt.
